# EUROPEAN PATENT SPECIFICATION

(11) **EP 2 813 209 B1**
(45) Date of publication and mention of the grant of the patent: **19.07.2017**
(21) Application number: 13746201.6
(22) Date of filing: 06.02.2013
(51) Int. Cl.: A61K 8/72, A61Q 19/00

(54) **HYDRATING COMPOSITION, USE OF A HYDRATING COMPOSITION AND COSMETIC FORMULATION CONTAINING A HYDRATING COMPOSITION**
HYDRIERUNGSZUSAMMENSETZUNG, VERWENDUNG EINER HYDRIERUNGSZUSAMMENSETZUNG UND KOSMETISCHE FORMULIERUNG MIT EINER HYDRIERUNGSZUSAMMENSETZUNG
COMPOSITION HYDRATANTE, UTILISATION D'UNE COMPOSITION HYDRATANTE ET FORMULATION COSMÉTIQUE CONTENANT UNE COMPOSITION HYDRATANTE

(30) Priority: 10.02.2012 BR 102012003136
(43) Date of publication of application: 17.12.2014
(73) Proprietor: Oxiteno S.A. Industria e Comercio, Sao Paulo - SP 01317-001 (BR)
(72) Inventor: OLIVEIRA, Lêda, Fernanda, de Jesus, Ipiranga - SP 04278-040 (BR); DA SILVA, André, Luis, Conde, São Caetano do Sul - SP 09560-500 (BR)
(74) Representative: Hörschler, Wolfram Johannes
(86) International application number: PCT/BR2013/000039
(87) International publication number: WO 2013/116914

(56) References cited:
- EP-A1- 1 374 847
- EP-A2- 0 396 405
- EP-A2- 1 284 136
- EP-A2- 1 284 136
- WO-A1-2005/037244
- WO-A1-2005/063848
- WO-A2-2009/015858
- US-A- 4 687 843
- US-A- 4 687 843

## Description

### Field of the Invention

The present invention relates to a hydrating composition for cosmetics. Specifically, the present invention describes a composition comprising a mixture of esters of one or more alkoxylated polyols that provides a capability to hydrate the skin, as well as a cosmetic formulation containing the said composition.

### Background of the Invention

The skin is formed of three layers, the most internal one being designated as the hypodermis, the intermediate layer being designated as the derma, and the outermost layer being designated as the epidermis. In order that the skin stays healthy, it is important that the upper layer of the epidermis, the Stratum Corneum [or *"horned layer"*], is kept hydrated. By skin hydration it is understood the relative increase of its hydric [*water-based moisture*] content, as determined by the corneometry test (U. Heinrich et al., "Multicentre comparison of skin hydration in terms of physical-, physiological- and product-dependent parameters by the capacitance method - Corneometer CM 825", International Journal of Cosmetic Science, 25, 45-51, 2003). In addition to providing flexibility to the skin and avoiding the drying out or parching thereof and the appearance of fissures of the skin or splitting or cracking thereof, hydration is also important in preventing the onset of chronic diseases.

The most common manner of hydrating the skin is by means of the use of hydrating cosmetic formulations. Such formulations are normally presented in the form of body butters, creams and lotions or milks for the skin, which basically differ from one another in terms of consistency. In general, those formulations constitute emulsions that may consist in oil in water emulsions (O/W), wherein the water constitutes the outermost phase of the emulsion, or water in oil emulsions (W/O), wherein the oil constitutes the outermost phase. Some formulations are also presented as micro-emulsions or nano-emulsions, evidencing increased stability.

The cosmetic formulations may be formed by a great variety of components, among which one or more (i) emollients, such as oils or silicones, which account for the lipidic replenishment of the skin; (ii) emulsifiers, commonly constituted by one or more surfactants, which main function is to provide stability to the formulation; (iii) consistency [*"skin-tightening"*] agents that may consist in gums or polymers used for viscosity adjustment; and (iv) other components, such as vitamins, antioxidants and hydration agents. Glycerin is the most commonly used compound as hydration agent in cosmetics (S. Verdier-Sévrain, F. Bonté, "Skin hydration: a review on its molecular mechanisms", Journal of Cosmetic Dermatology, 6, 75-82, 2007), however compounds of different chemical natures, among which certain polymers, may evidence this effect. Cosmetic formulations with hydrating action that contain polymers belonging to distinct chemical classes, such as polysaccharides, amino acids or synthetic polymers, have been devised (see documents Nos. US2004/0191205; US2007/0202070; L. Chrit et al., "In vitro and in vivo confocal raman study of human skin hydration: assessment of a new moisturizing agent, pMPC", Biopolymers, 85, 359-369, 2006).

Among the different types of polymers used in cosmetic formulations there may be pointed out the derivatives of alkoxylated polyols, which evidence low levels of toxicity, do not act as irritants to the skin and may provide other desirable characteristics for a cosmetic composition, among which an adequate consistency and a pleasant sensory sensation. However, apparently, the use of that polymer as a hydrating agent in cosmetics was not much exploited up to the present time. One example found consists in United States invention No. US 4,687,843 ("Esterified propoxylated glucose compositions", 1987), wherein is described the use of propoxylated methyl glucoside esters in cosmetics for the purpose of hydrating the skin. In patent US 4,687,843, the said compounds were obtained by combining between 5 and 50 mols of propylene oxide for every mol of methyl glucoside and subsequent reaction of the propoxylated derivative with saturated or unsaturated fatty acids; there being preferred the formation of diesters. However, taking into account the relatively low degree of propoxylation, it is not expected that the esters of the invention described in United States patent No. US 4,687,843 might evidence superior hydration or event thickening properties.

In the course of recent tests to verify the thickening power of a new composition of alkoxylated polyol esters, it was surprisingly observed that the sensory effect that was produced could constitute the result of a hydration action caused by those polymers, which fact was subsequently confirmed by means of specific corneometry tests. It was surprisingly confirmed that the alkoxylated polyols esters composition having been devised evidences the capacity to maintain the skin hydrated for up to 24 hours and is compatible with other ingredients commonly used in cosmetics, thereby enabling the use thereof in various cosmetic formulations, such as emulsions (W/O or O/W), micro-emulsions and nano-emulsions.

Therefore, it is an object of the present invention the provision of a new hydrating composition comprising a mixture of esters of an alkoxylated polyol. One other object of the present invention is the use of this new composition in cosmetic formulations for the purpose of increasing cutaneous ("*skin*") hydration and consequently improving other properties thereof, such as softness, gloss and flexibility. There also constitute objects of the present invention cosmetic formulations containing this new hydrating composition. The advantages of the hydrating composition according to the present invention shall become evident in the description that is provided below herein.

### Brief Description of the Invention

The present invention describes a new hydrating composition that comprises a mixture of esters of one or more alkoxylated polyols and finds applicability in the formulation of cosmetics for the skin. The composition according to the present invention may be presented in solid form or in liquid form, in this case further containing one or more surfactants, emollients and optionally water. The hydrating composition described in the present invention is compatible with a great variety of emollients and other ingredients used in cosmetic formulations, and evidenced being capable of maintaining the skin hydrated for 24 hours.

### Brief Description of the Drawings

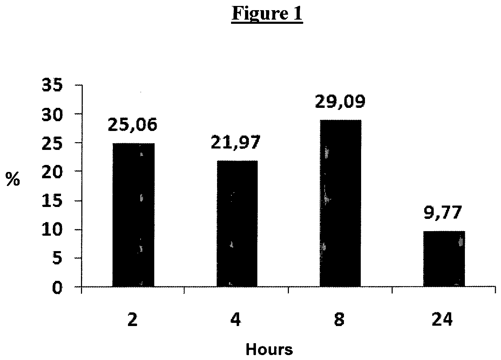

In Figure 1 there is shown the relative increase of skin hydration, expressed in percentage (%), as a function of time (hours), obtained as shown in Table 2.

### Detailed Description of the Invention

The hydrating composition to which the present invention refers comprises a mixture of esters of one or more alkoxylated polyols. The polyols deemed adequate for use in this invention comprise between 3 and 10 carbon atoms in total, and at least 3 free hydroxyls. Among the polyols that can be used there are included the glycerol and its derivatives, such as di- and triglycerol, in addition to pentaerythritol, neopentylglycol, trimethylolpropane, and sugar derivatives, such as mannitol, xylitol and sorbitol or a mixture of those polyols. For the adequate realization of this invention, the polyol should be alkoxylated until there are obtained more than 120 mols of the alkoxide per mol of polyol. The alkoxide may be ethylene oxide (EO), propylene oxide (PO), butylene oxide (BO) or a mixture of one or more of those oxides. The alkoxides react with the hydroxyls of the polyols leading to the formation of long polymer chains of the alkoxide that was employed, and may present themselves in the form of block copolymers or random ones when more than an alkoxide is used. The alkoxylated polyol is the esterified with one or more fatty acids that may have branched or straight chains, which may be saturated or unsaturated, and may contain between 8 and 22 carbon atoms. The esterification generates a mixture that may contain di-, tri-, tetra-, penta- and hexaesters of the alkoxylated polyol. The processes for obtainment of compounds of that type are already well established and have been described in United States patents Nos. US 4,983,329, US 5,464,874 and US 6,727,357, that have been cited herein as references.

In a preferred embodiment of the instant invention, the polyol that is used is sorbitol, which is alkoxylated with EO until evidencing more than 120 mols of EO for each mol of itself. Subsequently, the ethoxylated sorbitol is esterified with straight-chain saturated fatty acids, either in pure form or in the form of mixtures, in which connection those deemed more adequate are the caprylic acid, the capric acid, the lauric acid, the myristic acid, the palmitic acid, and preferably the stearic acid. There is thus obtained a composition comprising a mixture of ethoxylated sorbitol esters, wherein tetra-esters and penta-esters are in greater proportion in relation to the total esters based on the hydroxyl content and in the free acidity index of the composition, preferably adding up to more than 20% by weight of the total esters. Such composition is solid at ambient temperature and evidences a high hydrating power, and may be incorporated into various cosmetic formulations by heating, in the same manner practiced in connection with conventional formulations.

Both the handling of and the incorporation of the hydrating composition described herein into the various types of cosmetic formulations may be facilitated when the composition is in the liquid state. In that case, in order that the composition may still maintain a high hydrating power, there is recommended the addition of one or more surfactants, solvents, and optionally water. Thus, in another preferred embodiment of the present invention, the hydrating composition is presented in liquid form and comprises from 35 to 60% by weight of a mixture of esters of one or more alkoxylated polyols containing more than 120 mols of alkoxide for each mol of polyol, in addition to 25 to 40% by weight of esters of polyethylene glycols, 1 to 5% by weight of ethoxylated sorbitan esters, 0.5 to 2.0% by weight of glycols and, optionally, water up to 27% by weight. One example of the hydrating composition in the liquid form is presented in Table 1. Such composition can be conveniently used for the preparation of various cosmetic formulations, among which W/O emulsions, O/W emulsions, micro-emulsions, nano-emulsions ort any other type of cosmetic emulsion in the form of butter, cream, lotion or milk for the skin.

**Table 1: Example of a hydrating composition in liquid form.**

| **The percentages by weight are relative to the total weight of the composition.** | |
|---|---|
| COMPONENTS | PERCENTAGE BY WEIGHT |
| Mixture of esters of ethoxylated sorbitol | 50% |
| Polyethylene glycol esters | 21% |
| Ethoxylated sorbitan esters | 2% |
| Propylene glycol | 1% |
| Water (optional) | 26% |

The use of the composition according to the present invention in liquid form for hydrating the skin and consequently enhancing the smoothness thereof, its gloss and flexibility, evidences a number of advantages. For example, due to the chemical nature of the selected alkoxylated polyols the hydrating compositions in liquid form, such as that foreseen in Table 1, may be directly incorporated into the cosmetic formulations without requiring heating or vigorous stirring, as they homogenize easily. On an industrial scale, such operation is carried out in a tank provided with a stirring or agitating means and operating at ambient temperature. Thus, according to the present invention, a hydrating cosmetic formulation may be obtained by means of the mixture of one or more emulsifiers, adding up to a total of between 8 and 12% by weight of the formulation, one or more emollients also adding up to a total of between 8 to 12% by weight of the formulation, between 1 and 5% by weight of the hydrating composition of Table 1, and the remaining consisting in water. One example of a cosmetic formulation that comprises the hydrating composition provided in the present invention is presented in Table 2.

**Table 2: Example of a cosmetic formulation containing the hydrating composition of Table 1 (PPG15 = polypropylene glycol 15 stearyl ether).**

| COMPONENTS | CONCENTRATION (% by weight) |
|---|---|
| Ethoxylated castor bean oil (emulsifier) | 3.0% |
| Span 80 (emulsifier) | 7.0% |
| Mineral oil (emollient) | 4% |
| PPG 15 (emollient) | 6% |
| Hydrating composition of Table 1 | 3.0% |
| Water | q.s. *(sufficient amount to complete)* 100% |

One other advantage of the present invention is associated with the chemical structure of the alkoxylated polyols esters having been employed. The long polymer chains of the alkoxides, particularly of EO, form strong hydrogen bounds with the water molecules, thereby retaining moisture. However, the retained water molecules evidence lesser mobility, which leads to a thickening of the formulation. Therefore, the same mechanism that leads to hydration might also alter the viscosity of the formulation, which in such case will not require a thickener. In other words, in addition to lending hydrating power to a cosmetic formulation, the incorporation of the composition according to the present invention allows the control of the viscosity of the formulation, a fact which was not observed in previous inventions (US 4,687,843) and which is deemed to constitute a distinguishing factor in the present case.

The hydrating power of a cosmetic formulation may be easily confirmed by means of already well-established techniques, as is the case of corneometry (U. Heinrich, 2003, op. cit.), whereby is determined the hydric [*"water"*] content of the Stratum Corneum based on skin capacitance measurements. Corneometry tests were conducted with 20 voluntary subjects using two formulations: one which was exactly as described in Table 2, and one other that was similar, however not containing the hydrating composition of Table 1. Upon an initial conditioning of the volunteers for 60 minutes in a climate-controlled room, the study areas were defined to constitute a rectangle measuring 10 cm² on each forearm of the volunteers. For each of such volunteers, the formulation of Table 2 was applied to one of the forearms, while in the other forearm, which was used as control, there was applied the solution of Table 2 without the hydrating composition. There was used a Cutometer MPA 580 probe coupled to the equipment Multi Probe Adapter MPA 580 (CKeletronics, Germany). Measurements of 22+/-2 °C and relative humidity of 50+/-5 UR were conducted 2, 4, 8 and 24 hours after application of the composition on one forearm of each volunteer. In Figure 1 there is shown the average variation of the hydration percentage as a function of time, of the skin of the forearm of the 20 volunteers, obtained based on the difference between the measurements made on the area where the formulation containing the hydrating composition was applied, and those made on the forearm used as control, whereon was applied a formulation devoid of the hydrating composition. In Figure 1 the percentages represent, in average, how much more hydrated was the area that received the formulation containing the hydrating composition relatively to the control. It may be clearly observed that the hydration in the area whereon the formulation described in Table 2 was applied was higher than that of the control by more than 20% up to 8 hours upon application, whereupon it reached the maximum value of 29.09%. Even after 24 hours, the skin of the forearm whereon was applied the formulation containing the hydrating composition was still about 10% more hydrated than the skin of the forearm used as control.

Other observations showed that, in addition to not irritating the skin of the volunteers, the hydrating compositions obtained in accordance with the present invention present pleasant sensory properties, thus fulfilling the market requirements.

## Claims

1. A hydrating composition, **characterized by** comprising a mixture including di-, tri-, tetra-, penta- and hexa-esters of an alkoxylated polyol with more than 120 mols of the alkoxide for each mol of polyol, and which minimum content of tetra- and penta-esters corresponds preferably to 20% by weight of the total amount of esters.

2. A hydrating composition as claimed in claim 1, **characterized in that** the esters of the alkoxylated polyol are obtained from saturated or unsaturated fatty acids containing between 8 and 22 carbon atoms, preferably from stearic acid.

3. A hydrating composition as claimed in any one of claims 1 or 2, **characterized in that** the alkoxylated polyol is obtained by combining ethylene oxide, propylene oxide, butylene oxide or mixtures thereof with pentaerythritol, neopentyl glycol, trimethylolpropane, glycerol, one or more derivatives of glycerol, one or more derivatives of sugars, or mixtures thereof.

4. A hydrating composition as claimed in any one of claims 1 to 3, **characterized in that** the alkoxylated polyol is ethoxylated sorbitol, having more than 120 mols of ethylene oxide for each mol of sorbitol.

5. A hydrating composition as claimed in any one of claims 1 to 4, **characterized by** further comprising one or more solvents, surfactants, and optionally, water.

6. A hydrating composition as claimed in claim 5, **characterized in that** the solvents belong to the class of glycols and the surfactants are selected from among esters of polyethylene glycols, ethoxylated esters of sorbitan, or mixtures thereof.

7. A hydrating composition as claimed in any one of claims 5 or 6, **characterized by** comprising from 35 to 60% by weight of a mixture of esters of an alkoxylated polyol containing more than 120 mols of the alkoxide for each mol of the polyol, from 25 to 40% by weight of esters of polyethylene glycols, from 1 to 5% by weight of ethoxylated esters of sorbitan, from 0.5 to 2.0% by weight of glycols, and optionally, water up to 27% by weight.

8. The use of a hydrating composition as defined in any one of claims 1 to 7, **characterized by** occurring in the preparation of cosmetic formulations for hydrating the skin.

9. The use of a composition as claimed in claim 8, **characterized in that** the alkoxylated polyol is ethoxylated sorbitol and **in that** the esters are obtained preferably from stearic acid.

10. A cosmetic formulation containing a hydrating composition, **characterized by** comprising: (i) one or more emulsifiers adding up to a total of between 8 and 12% by weight of the formulation; (ii) one or more emollients adding up to a total of between 8 to 12% by weight of the formulation; (iii) between 1 and 5% by weight of a hydrating composition comprising from 35 to 60% by weight of a mixture of esters of an alkoxylated polyol containing more than 120 mols of the alkoxide for each mol of the polyol, from 25 to 40% by weight of esters of polyethylene glycols, from 1 to 5% by weight of ethoxylated esters of sorbitan, from 0.5 to 2.0% by weight of glycols, and optionally, water up to 27% by weight; and (iv) the remaining consisting in water.

11. A formulation, as claimed in claim 10, **characterized in that** the esters of the alkoxylated polyol are esters of ethoxylated sorbitol obtained from stearic acid.

12. A formulation, as claimed in claim 10, **characterized in that** the emulsifier comprises one or more ethoxylated vegetable oils, one or more derivatives of sorbitan, or mixtures obtained from among the same.

13. A formulation, as claimed in claim 10, **characterized in that** the emollient comprises one or more mineral oils, one or more derivatives of glycol, or mixtures obtained from among the same.

14. A formulation, as claimed in any one of claims 10 to 13, **characterized in that** the formulation is a W/O emulsion, an O/W emulsion, a micro-emulsion or a nano-emulsion.

## Patentansprüche

1. Hydratisierende Zusammensetzung, **dadurch gekennzeichnet, dass** diese ein Gemisch, enthaltend Di-, Tri-, Tetra-, Penta- und Hexa-Ester eines alkoxylierten Polyols mit mehr als 120 Mol Alkoxid, bezogen auf jedes Mol Polyol, umfasst und dass dessen Mindestgehalt an Tetra- und Penta-Estern vorzugsweise 20 Gew.-% der Gesamtmenge an Estern entspricht.

2. Hydratisierende Zusammensetzung wie in Anspruch 1 beansprucht, **dadurch gekennzeichnet, dass** die Ester des alkoxylierten Polyols aus gesättigten oder ungesättigten Fettsäuren, enthaltend zwischen 8 und 22 Kohlenstoffatomen, vorzugsweise aus Stearinsäure, erhalten werden.

3. Hydratisierende Zusammensetzung wie in einem der Ansprüche 1 oder 2 beansprucht, **dadurch gekennzeichnet, dass** das alkoxylierte Polyol durch Kombinieren von Ethylenoxid, Propylenoxid, Butylenoxid oder Gemischen davon mit Pentaerythritol, Neopentylglykol, Trimethylolpropan, Glycerin, einem oder mehreren Derivat(en) von Glycerin, einem oder mehreren Derivat(en) von Zuckern oder Gemischen davon, erhalten wird.

4. Hydratisierende Zusammensetzung wie in einem der Ansprüche 1 bis 3 beansprucht, **dadurch gekennzeichnet, dass** das alkoxylierte Polyol ethoxyliertes Sorbit mit mehr als 120 Mol Ethylenoxid, bezogen auf jedes Mol Sorbit, ist.

5. Hydratisierende Zusammensetzung wie in einem der Ansprüche 1 bis 4 beansprucht, **dadurch gekennzeichnet, dass** ferner ein oder mehrere Lösungsmittel, Tensid(e) und gegebenenfalls Wasser umfasst ist bzw. sind.

6. Hydratisierende Zusammensetzung wie in Anspruch 5 beansprucht, **dadurch gekennzeichnet, dass** die Lösungsmittel zur Klasse der Glykole gehören und die Tenside aus Estern von Polyethylenglykolen, ethoxylierten Estern von Sorbitan oder Gemischen davon, ausgewählt sind.

7. Hydratisierende Zusammensetzung wie in einem der Ansprüche 5 oder 6 beansprucht, **dadurch gekennzeichnet, dass** diese 35 bis 60 Gew.-% eines Gemisches von Estern eines alkoxylierten Polyols, enthaltend mehr als 120 Mol Alkoxid, bezogen auf jedes Mol des Polyols, 25 bis 40 Gew.-% Ester von Polyethylenglykolen, 1 bis 5 Gew.-% ethoxylierter Ester von Sorbitan, 0,5 bis 2,0 Gew.-% Glykole und gegebenenfalls bis zu 27 Gew.-% Wasser umfasst.

8. Verwendung einer hydratisierenden Zusammensetzung wie einem der Ansprüche 1 bis 7 definiert, **dadurch gekennzeichnet, dass** diese bei der Herstellung von kosmetischen Formulierungen zur Hydratisierung der Haut erfolgt.

9. Verwendung einer Zusammensetzung wie in Anspruch 8 beansprucht, **dadurch gekennzeichnet, dass** das alkoxylierte Polyol ethoxyliertes Sorbit ist und dass die Ester vorzugsweise aus Stearinsäure erhalten werden.

10. Kosmetische Formulierung enthaltend eine hydratisierende Zusammensetzung, **dadurch gekennzeichnet, dass** diese umfasst.: (i) einen oder mehrere Emulgator(en), die sich zu einer Gesamtmenge von 8 bis 12 Gew.-% der Formulierung addieren; (ii) einen oder mehrere Weichmacher, die sich bis zu einer Gesamtmenge von 8 bis 12 Gew.-% der Formulierung addieren; (iii) zwischen 1 und 5 Gew.-% einer hydratisierenden Zusammensetzung, umfassend 35 bis 60 Gew.-% eines Gemisches von Estern eines alkoxylierten Polyols, das mehr als 120 Mol Alkoxid, bezogen jedes Mol des Polyols, enthält, 25 bis 40 Gew.-% Ester von Polyethylenglykolen, 1 bis 5 Gew.-% ethoxylierter Ester von Sorbitan, 0,5 bis 2,0 Gew.-% Glykole und gegebenenfalls bis zu 27 Gew.-% Wasser; und (iv) der Rest aus Wasser besteht.

11. Formulierung wie in Anspruch 10 beansprucht, **dadurch gekennzeichnet, dass** die Ester des alkoxylierten Polyols Ester von ethoxyliertem Sorbit sind, das aus Stearinsäure erhalten wird.

12. Formulierung wie in Anspruch 10 beansprucht, **dadurch gekennzeichnet, dass** der Emulgator ein oder mehrere ethoxylierte Pflanzenöl(e), ein oder mehrere Derivat(e) von Sorbitan oder Gemische aus diesen umfasst.

13. Formulierung wie in Anspruch 10 beansprucht, **dadurch gekennzeichnet, dass** der Weichmacher ein oder mehrere Mineralöl(e), ein oder mehrere Derivat(e) von Glykol oder Gemische aus diesen umfasst.

14. Formulierung wie in einem der Ansprüche 10 bis 13 beansprucht, **dadurch gekennzeichnet, dass** die Formulierung eine W/O-Emulsion, eine O/W-Emulsion, eine Mikroemulsion oder eine Nanoemulsion ist.

## Revendications

1. Composition hydratante, **caractérisée en ce qu'**elle comprend un mélange comportant des di-, tri-, tétra-, penta- et hexa-esters d'un polyol alcoxylé ayant plus de 120 moles de l'alcoxyde pour chaque mole de polyol, et dont la teneur minimale en tétra- et penta-esters correspond préférablement à 20% en poids de la quantité totale d'esters.

2. Composition hydratante selon la revendication 1, **caractérisée en ce que** les esters du polyol alcoxylé sont obtenus à partir d'acides gras saturés ou insaturés contenant entre 8 et 22 atomes de carbone, préférablement à partir d'acide stéarique.

3. Composition hydratante selon l'une quelconque des revendications 1 ou 2, **caractérisée en ce que** le polyol alcoxylé est obtenu par la combinaison d'oxyde d'éthylène, d'oxyde de propylène, d'oxyde de butylène ou de mélanges de ceux-ci avec du pentaérythritol, du néopentyl glycol, du triméthylolpropane, du glycérol, un ou plusieurs dérivés de glycérol, un ou plusieurs dérivés de sucres, ou des mélanges de ceux-ci.

4. Composition hydratante selon l'une quelconque des revendications 1 à 3, **caractérisée en ce que** le polyol alcoxylé est du sorbitol éthoxylé, ayant plus de 120 moles d'oxyde d'éthylène pour chaque mole de sorbitol.

5. Composition hydratante selon l'une quelconque des revendications 1 à 4, **caractérisée en ce qu'**elle comprend en outre un ou plusieurs solvants, agents tensioactifs, et éventuellement de l'eau.

6. Composition hydratante selon la revendication 5, **caractérisée en ce que** les solvants appartiennent à la classe des glycols et les agents tensioactifs sont choisis parmi les esters de polyéthylène glycols, les esters éthoxylés de sorbitane, ou des mélanges de ceux-ci.

7. Composition hydratante selon l'une quelconque des revendications 5 ou 6, **caractérisée en ce qu'**elle comprend de 35 à 60% en poids d'un mélange d'esters d'un polyol alcoxylé contenant plus de 120 moles de l'alcoxyde pour chaque mole du polyol, de 25 à 40% en poids d'esters de polyéthylène glycols, de 1 à 5% en poids d'esters éthoxylés de sorbitane, de 0,5 à 2,0% en poids de glycols, et éventuellement de l'eau jusqu'à 27% en poids.

8. Utilisation d'une composition hydratante telle que définie selon l'une quelconque des revendications 1 à 7, **caractérisée par** sa présence dans la préparation de formulations cosmétiques destinées à hydrater la peau.

9. Utilisation d'une composition selon la revendication 8, **caractérisée en ce que** le polyol alcoxylé est du sorbitol éthoxylé et **en ce que** les esters sont obtenus préférablement à partir d'acide stéarique.

10. Formulation cosmétique contenant une composition hydratante, **caractérisée en ce qu'**elle comprend : (i) un ou plusieurs émulsifiants constituant un total compris entre 8 et 12% en poids de la formulation ; (ii) un ou plusieurs émollients constituant un total compris entre 8 et 12% en poids de la formulation ; (iii) entre 1 et 5% en poids d'une composition hydratante comprenant de 35 à 60% en poids d'un mélange d'esters d'un polyol alcoxylé contenant plus de 120 moles de l'alcoxyde pour chaque mole du polyol, de 25 à 40% en poids d'esters de polyéthylène glycols, de 1 à 5% en poids d'esters éthoxylés de sorbitane, de 0,5 à 2,0% en poids de glycols, et éventuellement de l'eau jusqu'à 27% en poids ; et (iv) le complément étant constitué d'eau.

11. Formulation selon la revendication 10, **caractérisée en ce que** les esters du polyol alcoxylé sont des esters de sorbitol éthoxylé obtenus à partir d'acide stéarique.

12. Formulation selon la revendication 10, **caractérisée en ce que** l'émulsifiant comprend une ou plusieurs huiles végétales éthoxylées, un ou plusieurs dérivés de sorbitane, ou des mélanges obtenus parmi ceux-ci.

13. Formulation selon la revendication 10, **caractérisée en ce que** l'émollient comprend une ou plusieurs huiles minérales, un ou plusieurs dérivés de glycol, ou des mélanges obtenus parmi ceux-ci.

14. Formulation selon l'une quelconque des revendications 10 à 13, **caractérisée en ce que** la formulation est une émulsion E/H, une émulsion H/E, une microémulsion ou une nano-émulsion.
